(19) Europäisches Patentamt
European Patent Office
Office européen des brevets

(11) Veröffentlichungsnummer: **0 277 462 B1**

(12) **EUROPÄISCHE PATENTSCHRIFT**

(45) Veröffentlichungstag der Patentschrift: **29.04.92**

(51) Int. Cl.$^5$: **A61K 37/30**, A61K 9/08

(21) Anmeldenummer: **87810769.7**

(22) Anmeldetag: **18.12.87**

Die Akte enthält technische Angaben, die nach dem Eingang der Anmeldung eingereicht wurden und die nicht in dieser Patentschrift enthalten sind.

# (54) Verfahren zur Herstellung nasaler Lösungen enthaltend synthetisches Human calcitonin.

(30) Priorität: **23.12.86 CH 5200/86**

(43) Veröffentlichungstag der Anmeldung:
**10.08.88 Patentblatt 88/32**

(45) Bekanntmachung des Hinweises auf die Patenterteilung:
**29.04.92 Patentblatt 92/18**

(84) Benannte Vertragsstaaten:
**AT BE CH DE ES FR GB GR IT LI LU NL SE**

(56) Entgegenhaltungen:
**EP-A- 0 115 627**
**EP-A- 0 183 527**
**EP-A- 0 187 361**
**EP-A- 0 193 372**
**FR-A- 2 312 260**
**GB-A- 2 142 335**
**GB-A- 2 176 105**

**CHEMICAL ABSTRACTS, Band 105, 1986, Seite 375, Zusammenfassung Nr. 120813a, Columbus, Ohio, US; & JP-A-61 126 014 (TEIJIN LTD.) 13-06-1986**

(73) Patentinhaber: **CIBA-GEIGY AG**
**Klybeckstrasse 141**
**CH-4002 Basel(CH)**

(72) Erfinder: **Geller, Leo, Dr.**
**Rudolf Wackernagel-Strasse 14**
**CH-4125 Riehen(CH)**
Erfinder: **Glanzmann, Peter**
**J.J. Balmerstrasse 7**
**CH-4053 Basel(CH)**

**EP 0 277 462 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von pharmazeutischen Zusammensetzungen in Form von wässrigen Lösungen für die Verabreichung von synthetischem, humanem Calcitonin.

Calcitonine gehören zur Wirkstoffgruppe der Nebenschilddrüsenhormone und bestehen aus langkettigen Polypetiden mit unterschiedlicher Aktivität. Bestimmte Calcitonine, z.B. Human-, Salm- und Aalcalcitonin, sind synthetisch herstellbar, kommerziell erhältlich und allgemein für die Behandlung diverser Krankheiten verwendbar, z.B. Pagetsche Krankheit, Hypercalcämie oder Osteoporose.

Wie auch bei anderen Polypetiden, z.B. Insulin, sind enterale Verabreichungsformen problematisch. So werden Calcitonine schnell abgebaut und nur langsam von Körperflüssigkeiten aufgenommen. Daher waren bisher für solche Wirkstoffe nur parenterale Verabreichungsformen üblich. Diese Verabreichungsformen, insbesondere i.m. oder i.v., sind auch problematisch, da sie sich nur bedingt für die Selbstmedikation eignen und schmerzhaft sein können. Daher besteht das Bedürfnis nach bequemeren Verabreichungsformen, welche dem Patienten eine leicht handzuhabende Selbstmedikation mit einer der effektiven klinischen Behandlung entsprechenden Bioverfügbarkeit des Wirkstoffs ermöglichen.

In der Deutschen Offenlegungsschrift DE-A-3,335,086 sind Nasalpräparate, z.B. Tropfen und Sprays, für die Applikation von Calcitonin beschrieben. Generell haben Tropfen und Sprays bei der nasalen Applikation den Nachteil, dass die Flüssigkeit zu schnell abläuft.

In der U.S. Patentschrift 4,294,829 wird vorgeschlagen, statt Tropfen oder Sprays pulverförmige Mischungen von Calcitonin mit Methylcellulose in die Nase zu applizieren. Generell ist die nasale Verwendung von Pulvern wegen der Reizwirkung besonders auf trockene Schleimhäute nachteilig. Ausserdem lassen sich Pulver relativ ungenau dosieren, wenn sie nasal beispielsweise mit einer Sprühvorrichtung appliziert werden müssen.

In der Europäischen Patentanmeldung 183 527 sind intranasale Calcitoninformulierungen beschrieben, welche so unterschiedliche Absorptionsmittel wie Benzylalkohol, Ethanol, Thiamine, Salicylsäure, Capronsäure, Polyethylenglycole, Pyridoxal, Malonsäure und Pyrophosphorsäure. enthalten. Methyl- und Methylhydroxypropylcellulose sind in Kombination mit diesen Absorptionsmitteln als Hilfsstoffe ohne Angabe einer exakten Rezeptur genannt.

In der Britischen Patentanmeldung 2 142 335 werden intranasale, ölige Formulierungen von Derivaten des Calcitonins beschrieben. Zwar wird der Hilfsstoff Carboxymethylcellulose neben anderen Quellmitteln genannt, doch lassen sich aus dieser Publikation keine nasalen, wässrigen Formulierungen mit diesen Hilfsstoffen ableiten.

In der Französischen Patentanmeldung 2 312 260 sind Formulierungen mit Humancalcitonin beschrieben. Carboxymethylcellulose wird mit seiner bekannten Eigenschaft als Hilfsstoff zur Erhöhung der Viskosität genannt. Es fehlt aber ein Hinweis auf die nasale Applikation. Die mit vollständigen Rezepturen beschriebenen Suspensionen und Lösungen sind nur für die intramuskuläre und subkutane Applikation vorgesehen.

In der Europäischen Patentanmeldung 115 627 sind intranasale Calcitoninformulierungen beschrieben. Das Calcitonin selbst wird nicht weiter spezifiziert. Als Absorptionsmittel sind Tenside genannt. Zusätzlich zu den Tensiden können Quellmittel des Typs Celluloseether zugesetzt werden.

In der publizierten japanischen Patentanmeldung Sho-61-126014 sind viskose wässrige Lösungen mit Calcitonin, z.B. Salmcalcitonin, beschrieben, welche Hydroxypropylcellulose als Quellmittel enthalten. Bei Verwendung von Humancalcitonin sind diese durch Zufügen des Calcitonins zu einer Hydroxypropylcellulose-Lösung erhältlichen Lösungen nachteilig: In Helvetica Chimica Acta (P. Sieber et al.) Vol. 53, Fasc. 8 (1970), Nr. 255, SS. 2135-2150, insbesondere SS. 2141-2142, ist die Bildung von Assoziaten, insbesondere von Fibrillen, von Humancalcitonin in wässriger Lösung beschrieben. Solche Assoziate vermindern die Resorptionsfähigkeit von Humancalcitonin.

Es wurde überraschenderweise gefunden, dass sich durch Sterilfiltrieren einer wässrigen Lösung von synthetischem Humancalcitonin, Lyophilisieren und Auflösen des Lyophilisats in wässriger Lösung die Bildung von Fibrillen vermeiden lässt und pharmazeutische Zusammensetzungen in Form einer wässrigen Lösung für die nasale Verabreichung von synthetischem Humancalcitonin in gut dosierbaren Mengen herstellen lassen. Die vorliegende Erfindung betrifft daher ein Verfahren zur Herstellung von intranasal applizierbaren, wässrigen Lösungen von Humancalcitonin enthaltend

a) eine therapeutisch wirksame Menge von synthetischem Humancalcitonin;

b) Methylcellulose oder Methylhydroxypropylcellulose als viskositätserhöhende Quellmittel;

c) Trägerflüssigkeit mit gegebenenfalls weiteren Hilfsstoffen zur Herstellung von isotonischen Bedingungen, dadurch gekennzeichnet, dass man synthetisches Humancalcitonin in Wasser, gegebenenfalls unter Zusatz der Hilfsstoffe zur Herstellung der isotonischen Bedingungen, löst, die Lösung auf einen pH-Wert von 4-6 bringt, sterilfiltriert, ein Lyophilisat herstellt und dieses zur Trägerflüssigkeit enthaltend Methylcellulose oder Methylhydroxypropylcellulose hinzufügt.

Die weiter vorn und im folgenden genannten allgemeinen Begriffe haben im Rahmen der Beschreibung der vorliegenden Erfindung vorzugsweise die folgenden Bedeutungen:

Methylcellulose und Methylhydroxypropylcellulose haben einen durchschnittlichen molaren Substitutionsgrad (MS) grösser als eins und kleiner als drei und einen durchschnittlichen Polymerisationsgrad von 100-5000.

Der Substitutionsgrad ist ein Mass für die Substitution der Hydroxygruppen durch Niederalkoxygruppen (hier: Methoxygruppen) pro Glucoseeinheit. Der durchschnittliche molare Substitutionsgrad (MS) ist ein gemittelter Wert und gibt die Anzahl der Niederalkoxygruppen (hier: Methoxygruppen) pro Glucoseeinheit im Polymerisat an.

Der durchschnittliche Polymerisationsgrad (DP) ist ebenfalls ein gemittelter Wert und gibt die durchschnittliche Anzahl an Glucoseeinheiten im Cellulosepolymerisat an.

Bevorzugt ist Methylcellulose (DP: 200-1000, MS: 1,4-2,0) und Methylhydroxypropylcellulose (DP: 200-1000, MS: 1,4-2,0).

Die Trägerflüssigkeit hat einen pH-Wert kleiner als 6, der sich nach Auflösen der festgelegten Wirkstoffmenge des Calcitoninlyophilisats in der vorgesehenen Flüssigkeitsmenge einstellt. Der pH-Wert kann nach Auflösung des Wirkstoffs in der Trägerflüssigkeit zwischen 3 und 6 betragen.

Die Trägerflüssigkeit enthält vorzugsweise Zusätze zur Herstellung von isotonischen Bedingungen, z.B. ionische Zusätze wie Kochsalz, oder nichtionische Zusätze wie Sorbit, Mannit oder Glucose (Gerüstbildner) und zwar in der zur Herstellung von isotonischen Lösungen üblichen Konzentration, welche z.B. in "Hager" Band VIIa SS. 225-239 angegeben ist. Insbesondere wird als Trägerflüssigkeit Calciumfreie, isotonische Kochsalz- oder Sorbitlösung verwendet.

Die Trägerflüssigkeit kann ausserdem weitere pharmazeutisch annehmbare Zusatzstoffe enthalten, z.B. Konservierungsmittel wie Benzalkoniumchlorid, oder oberflächenaktive Mittel zur Verbesserung des Fliessverhaltens, insbesondere nicht-ionische Tenside aus der Gruppe Polyoxyalkylenäther höherer Alkohole, z.B. der allgemeinen Formel

$$RO-[(CH_2)_n-O]_x-H$$

worin RO der hydrophobe Rest eines höheren Alkohols, z.B. Lauryl- oder Cetylalkohol, eines Alkylphenols, oder eines Sterols, z.B. Lanosterol, Dihydrocholesterol oder Cholesterol, ist, sowie Mischungen von zwei oder mehreren solchen Aethern. Bevorzugte Polyoxyalkylenäther sind Polyoxyäthylen- und Polyoxypropylenäther mit hydrophoben Gruppen (d.h. worin n in der o.e. Formel 2 oder 3 ist), besonders Lauryl-, Cetyl- und Cholesterolpolyoxyäthylen- und -polyoxypropylenäther, sowie Mischungen von zwei oder mehreren solchen Aethern.

Die Hydroxygruppen am endständigen Alkylenrest dieser oben erwähnten Polyäther können teilweise oder vollständig acyliert sein, z.B. mit Acylresten aliphatischer Carbonsäuren, wie z.B. Essigsäure.

Bevorzugte Polyäther haben ein hydrophil-lipophil Verhältnis (HLB-Gruppennummer) von 10 bis 20, speziell von 12 bis 16.

Besonders geeignete Polyäther haben einen Mittelwert der sich wiederholenden Einheiten im Polyoxyalkylenteil (Klammerausdruck in der obigen Formel) zwischen 4 und 75, besonders zwischen 8 und 30 und ganz besonders zwischen 16 und 26. Die Polyäther können gemäss bekannten Methoden erhalten werden. Eine grosse Auswahl solcher Produkte steht kommerziell zur Verfügung und wird z.B. von der Firma Amerchol unter dem Markennamen Solulan®, von den Firmen KAO Soap, ICI und Atlas unter dem Markennamen Emalex®, Brij® und Laureth® und von der Firma Croda unter dem Markennamen Cetomacrogol® verkauft.

Geeignete oberflächenaktive Mittel sind ausserdem insbesondere nichtionische Tenside vom Typ Fettsäure-Polyhydroxyalkoholester wie Sorbitanmonolaurat, -oleat, -stearat oder -palmitat, Sorbitantristearat oder -trioleat, Polyoxyäthylen-Addukte von Fettsäure-Polyhydroxyalkoholestern wie Polyoxyäthylen-sorbitanmonolaurat, -oleat, -stearat, -palmitat, -tristearat oder -trioleat, Polyäthylenglycol-Fettsäureesterwie Polyoxyäthylstearat, Polyäthylenglycol-400-stearat, Polyäthylenglycol-2000-stearat, insbesondere AethylenoxidPropylenoxid Blockpolymere vom Typ Pluronics® (Wyandotte).

Die Trägerflüssigkeit kann noch weitere pharmazeutisch annehmbare Zusatzstoffe enthalten, z.B. ätherische Oele zur Verbesserung des Geruchs, z.B. Menthol, Paraffin oder Glycerin zur Verbesserung des Fliessverhaltens, Zucker und/oder Süssstoffe zur Verbesserung des Geschmacks und Aromastoffe.

Die nasale Applikation der pharmazeutischen Zusammensetzungen ermöglicht eine komfortable und einfache Verabreichung von Humancalcitonin durch den Patienten selbst, so dass die bisher übliche parenterale Verabreichung durch den behandelnden Arzt entfallen kann.

Die pharmazeutischen Zusammensetzungen zeichnen sich durch gute Verträglichkeit und lange Verweildauer des Wirkstoffs am Anwendungsort aus. Die hohe Viskosität der zu applizierenden wässrigen Lösung bewirkt eine lange Verweildauer auf den Schleimhäuten und damit eine besonders gute Resorption des Wirkstoffs.

Die nasal zu applizierende wässrige Lösung, enthaltend synthetisches Humancalcitonin, senkt den Plasma-Calcium- und den Plasma-Phosphatspiegel im Blut von Warmblütern (Menschen und Tiere) und kann daher zur Behandlung von Hypercalcämie und/oder Knochenkrankheiten wie Pagetsche Krankheit oder Osteoporose verwendet werden.

Die zu applizierende Dosis Humancalcitonin und die Konzentration des Wirkstoffs in der pharmazeutischen Zusammensetzung hängt von der zu behandelnden Krankheit ab und dem Zustand des Patienten.

Die Resorption von Humancalcitonin (bestimmbar als Blutplasma-Konzentration), ist nach nasaler Verabreichung überraschend hoch. Sie kann sogar höhere Werte als entsprechende Werte erreichen, welche man nach intramuskulärer Injektion bestimmt. Die zu verabreichende Dosismenge kann ein Mehrfaches der bekannten Dosismengen betragen, welche z.B. bei intramuskulärer Verabreichung üblich sind.

Bisher wurden bei s.c. oder i.m. Injektionen von Humancalcitonin, Einzeldosen von ca. 0,5 mg Wirkstoff 1x täglich bis 3x in der Woche verabreicht. Die erfindungsgemässe nasale Verabreichung wird im Behandlungszeitraum Dosen von 1,0-10,0 mg bei einer Frequenz von 1x täglich bis 3x in der Woche, erfordern. Man kann die oben erwähnten Dosen bei einer einzigen Verabreichung applizieren, d.h. bei der Behandlung werden nasale Einzeldosen, enthaltend 1,0-10,0 mg synthetisches Humancalcitonin verabreicht. Als Alternative können diese Dosismengen auch über 2 bis 4 Verabreichungen pro Tag verteilt werden. Das Gesamtvolumen der Zusammensetzung für die nasale Verabreichung beträgt vorzugsweise 0,1 bis 0,5 ml.

Die vorliegende Erfindung betrifft vorzugsweise ein Verfahren zur Herstellung einer pharmazeutischen Zusammensetzung in Form einer wässrigen Lösung für die nasale Applikation von synthetischem Humancalcitonin mit einem Viskositätsbereich von 20-300 mPasec (25 °C). Diese pharmazeutische Zusammensetzung enthält bevorzugt:

a) die therapeutisch wirksame Menge von synthetischem Humancalcitonin,
b) 0,2-3,0 Gew.% Methylcellulose oder Methylhydroxypropylcellulose (DP: 200-1000, MS: 1,4-2,0) und
c) die Trägerflüssigkeit mit Hilfsstoffen zur Herstellung von isotonischen Bedingungen.

Die vorliegende Erfindung betrifft in erster Linie ein Verfahren zur Herstellung einer pharmazeutischen Zusammensetzung in Form einer wässrigen Lösung für die nasale Applikation von synthetischem Humancalcitonin mit einem Viskositätsbereich von 20-100 mPasec (25 °C). Diese pharmazeutische Zusammensetzung enthält:

a) die therapeutisch wirksame Menge von synthetischem Humancalcitonin,
b) 0,5-1,0 Gew.% Methylcellulose oder Methylhydroxypropylcellulose (DP: 200 bis 1000, MS: 1,4-2,0) und
c) die Trägerflüssigkeit mit Hilfsstoffen zur Herstellung von isotonischen Bedingungen.

In einer bevorzugten Ausführungsform des Verfahrens stellt man das Lyophilisat des synthetischen Humancalcitonins her, z.B. indem man das Calcitonin mit der erforderlichen Menge an nichtionischen Zusatzstoffen zur Herstellung von isotonischen Bedingungen (Gerüstbildner), z.B. Mannit, in dest. Wasser löst, die Lösung mit verdünnter wässriger Base, z.B. Natronlauge, auf einen pH von 4-6 bringt, sterilfiltriert und anschliessend lyophilisiert. Das wirkstoffhaltige Lyophilisat wird zu einer Lösung (Schleim) enthaltend Methylhydroxypropylcellulose oder Methylcellulose und gegebenenfalls weitere Zusätze wie Sorbit und Konservierungsmittel wie Benzalkoniumchlorid gegeben und gelöst. Diese schleimartige Lösung kann sich der Patient mittels Tropfpipette in die Nase applizieren.

Die pharmazeutischen Zusammensetzungen sind vorzugsweise isotonisch, wobei der osmotische Druck zwischen 260 und 380 mOsm/kg variieren kann.

Der gewünschte Viskositätsbereich kann durch Zugabe geeigneter Mengen der Komponente b) eingestellt werden. Bei zu niedriger Viskosität läuft die Flüssigkeit zu schnell ab. Bei zu hoher Viskosität wird die Flüssigkeit zäh und lässt sich schlecht applizieren. Einen besonders geeigneten Viskositätsbereich besitzen wässrige Lösungen mit 0,2-3 % Methylcellulose oder Methylhydroxypropylcellulose (DP: 200-1000, MS: 1,4-2,0). Die entsprechenden Viskositäten liegen bei 20 °C in der Grössenordnung von 5-5000 mPasec, insbesondere 20-300 mPasec, und vor allem 20-100 mPasec (25 °C). Auf die in Hager Band VII S. 115-118

angegebenen genauen Vorschriften, worin die Herstellung von wässrigen Lösungen gewünschter Viskosität in Abhängigkeit von der Konzentration des betreffenden viskositätserhöhenden Quellmittels beschrieben ist, wird verwiesen. Durch Zugabe des Wirkstoffs, Salzen, oberflächenaktiven Mitteln und anderen Zusätzen können sich die Viskositätswerte der reinen Lösungen mit Quellmitteln verändern. Generell müssen die viskosen Lösungen genügendes Fliessvermögen besitzen und eine ausreichende Benetzung der Nasenschleimhäute gewährleisten.

Es können die erfindungsgemässen pharmazeutischen Präparate verabreicht werden, welche die genannten Komponenten synthetisches Humancalcitonin a) und Quellmittel b) nebst Zusätzen gelöst enthalten. Diese flüssige Darreichungsform kann dem Patienten nasal in üblicher Weise mit einer Tropfpipette verabreicht werden. Der Patient kann sich die Lösung in der vorgesehenen Menge selbst verabreichen. Die pharmazeutischen Zusammensetzungen können auch in-situ zubereitet werden, z.B. indem man eine zuvor hergestellte Lösung ohne Wirkstoff aber mit Quellmittel und Zusatzstoffen, z.B. mit Methylcellulose oder Methylhydroxypropylcellulose und Sorbit oder Natriumchlorid, vor der Anwendung mit einem Lyophilisat des Calcitonins versetzt und die Mischung anschliessend nasal appliziert.

Die folgenden Beispiele illustrieren die Operabilität der Erfindung ohne diese zu beschränken. Temperaturen sind in Graden Celsius angegeben.

Beispiel 1:

a) Herstellung des Wirkstofflyophilisates

| Komponenten | |
|---|---|
| CIBACALCIN® | 10.0 mg |
| Mannit | 10.0 mg |

10 mg CIBACALCIN (synthetisches Humancalcitonin) und 10 mg Mannit werden bei Raumtemperatur unter Stickstoff in 2.0 ml dest. Wasser gelöst. Der pH-Wert wird mit ca. 4 mg 1N wässriger NaOH auf 4.5 gestellt. Die Lösung wird durch ein steriles Membranfilter (0.2 μm Porengrösse) sterilfiltriert und die sterile Lösung in eine sterile Glasviale abgefüllt. Die Viale wird bei -40° eingefroren und in einer Gefriertrocknungsapparatur lyophilisiert.

b) Herstellung der quellmittelhaltigen Lösung

| Komponenten | |
|---|---|
| Methocel® 90 HG 4000 cP | 50.0 mg |
| Sorbit | 500.0 mg |
| Benzalkoniumchlorid | 1.0 mg |
| Dest. Wasser | 10.0 ml |

Methylhydroxypropylcellulose (METHOCEL®), Sorbit und Benzalkoniumchlorid werden in dest. Wasser vermischt. Man lässt mehrere Stunden bei 5° quellen und filtriert danach die viskose Lösung durch einen Scrynel®Filter mit einer Porengrösse von 10 μm. Die filtrierte Lösung wird in eine Glasviale abgefüllt und sterilisiert, z.B. in einem Autoklaven. Der pH-Wert der sterilisierten Lösung liegt bei pH 6-7.

c) Herstellung und Applikation der nasalen CIBACALCIN-Lösung (2.0 mg Cibacalcinlyophilisat/0.4 ml quellmittelhaltige Lösung)

1 Viale mit gemäss Beispiel 1 a) hergestelltem Wirkstoff-Lyophilisat wird in 2.0 ml gemäss Beispiel 1 b) hergestelltem quellmittelhaltigem Lösungsmittel aufgelöst. Der pH-Wert der Lösung entspricht demjenigen des Lyophilisates. 0.4 ml der schleimigen CIBACALCIN-Lösung werden mit einer Tropfpipette dem liegenden Patienten in die Nase (beide Nasenlöcher) appliziert.

Beispiel 2:

a) Analog Beispiel 1 a) lassen sich Wirkstofflyophilisate aus 10 mg CIBACALCIN (ohne Mannitzusatz) und 10 mg CIBACALCIN mit 30 mg Mannit herstellen, wobei man den pH-Wert der zu lyophilisierenden Lösung mit verdünnter, wässriger Natronlauge auf pH 3,5 bis 6,0 einstellt.
b) Analog Beispiel 1 b) lassen sich quellmittelhaltige Lösungen aus 20 bis 100 mg Methylhydroxypropylcellulose (METHOCEL®), 500 mg Sorbit und 1,0 mg Benzalkoniumchlorid herstellen.
c) Analog Beispiel 1 c) wird ein Wirkstofflyophilisat mit den in Beispiel 2 a) genannten Zusammensetzungen mit einer quellmittelhaltigen Lösung mit den in Beispiel 2 b) angegebenen Zusammensetzungen versetzt und nasal appliziert.

## Patentansprüche

**1.** Verfahren zur Herstellung Von intranasal applizierbaren, wässrigen Lösungen von Humancalcitonin enthaltend:
a) eine therapeutisch wirksame Menge von synthetischem Humancalcitonin;
b) Methylcellulose oder Methylhydroxypropylcellulose als viskositätserhöhende Quellmittel;
c) Trägerflüssigkeit mit gegebenenfalls weiteren Hilfsstoffen zur Herstellung von isotonischen Bedingungen, dadurch gekennzeichnet, dass man synthetisches Humancalcitonin in Wasser, gegebenenfalls unter Zusatz der Hilfsstoffe zur Herstellung der isotonischen Bedingungen, löst, die Lösung auf einen pH-Wert von 4-6 bringt, sterilfiltriert, ein Lyophilisat herstellt und dieses zur Träger flüssigkeit enthaltend Methylcellulose oder Methylhydroxypropylcellulose hinzufügt.

**2.** Verfahren gemäss Anspruch 1, dadurch gekennzeichnet, dass man synthetisches Humancalcitonin in Wasser enthaltend Mannit als Hilfsstoff zur Herstellung von isotonischen Bedingungen löst.

**3.** Verfahren gemäss Anspruch 1 oder 2, dadurch gekennzeichnet, dass man das Lyophilisat zur Trägerflüssigkeit enthaltend 0,2-3,0 Gew.-% Methylcellulose oder Methylhydroxypropylcellulose hinzufügt.

**4.** Verfahren gemäss Anspruch 1 oder 2, dadurch gekennzeichnet, dass man das Lyophilisat zur Trägerflüssigkeit enthaltend 0,5-1,0 Gew.-% Methylcellulose oder Methylhydroxypropylcellulose hinzufügt.

## Claims

**1.** A process for the preparation of intranasally administrable aqueous solutions of human calcitonin comprising:
a) a therapeutically effective amount of synthetic human calcitonin;
b) methylcellulose or methylhydroxypropylcellulose as viscosity-enhancing swelling agent;
c) a carrier liquid containing, optionally, further excipients for producing isotonic conditions, wherein synthetic human calcitonin is dissolved in water, optionally with the addition of excipients to produce isotonic conditions, the solution is adjusted to a pH value of 4-6 and filtered under sterile conditions, a lyophilisate is produced and this is added to the carrier liquid containing methylcellulose or methylhydroxypropylcellulose.

**2.** A process according to claim 1, wherein synthetic human calcitonin is dissolved in water containing mannitol as excipient for producing isotonic conditions.

**3.** A process according to either claim 1 or claim 2, wherein the lyophilisate is added to the carrier liquid containing 0.2-3.0 % by weight of methylcellulose or methylhydroxypropylcellulose.

**4.** A process according to either claim 1 or claim 2, wherein the lyophilisate is added to the carrier liquid containing 0.5 to 1.0 % by weight of methylcellulose or methylhydroxypropylcellulose.

## Revendications

1. Procédé de préparation de solutions aqueuses de calcitonine humaine, aptes à l'administration intranasale, contenant :
   a) une quantité thérapeutique efficace de calcitonine humaine synthétique;
   b) de la méthylcellulose ou de la méthylhydroxypropylcellulose en tant qu'agent gonflant accroissant la viscosité;
   c) un liquide véhicule contenant le cas échéant d'autres produits auxiliaires servant à établir des conditions isotoniques, ce procédé se caractérisant en ce que l'on dissout de la calcitonine humaine synthétique dans de l'eau, éventuellement avec adjonction des produits auxiliaires nécessaires pour établir les conditions isotoniques, on règle la solution à un pH de 4 à 6, on en prépare un lyophilisat et on ajoute ce lyophilisat au liquide véhicule contenant la méthylcellulose ou la méthylhydroxypropylcellulose.

2. Procédé selon la revendication 1, caractérisé en ce que l'on dissout la calcitonine humaine synthétique dans de l'eau contenant du mannitol qui sert de produit auxiliaire pour l'établissement des conditions isotoniques.

3. Procédé selon la revendication 1 ou 2, caractérisé en ce que l'on ajoute le lyophilisat au liquide véhicule contenant 0,2 à 3,0 % en poids de méthylcellulose ou de méthylhydroxypropylcellulose.

4. Procédé selon la revendication 1 ou 2, caractérisé en ce que l'on ajoute le lyophilisat au liquide véhicule contenant 0,5 à 1,0 % en poids de méthylcellulose ou de méthylhydroxypropylcellulose.